# NEW EUROPEAN PATENT SPECIFICATION

(11) **EP 0 403 187 B2**
(45) Date of publication and mention of the opposition decision: **29.12.1999**
(45) Mention of the grant of the patent: 14.02.1996
(21) Application number: 90306292.5
(22) Date of filing: 08.06.1990
(51) Int. Cl.: B32B 5/18, D04H 13/00, A61F 13/15

(54) **Flexible composite with a perforated plastics film bonded to a fibrous layer and method of making the same**
Flexibles Verbundmaterial mit einer an einer Faserschicht angeklebten perforierten Kunststoffolie sowie Verfahren zur Herstellung desselben
Matériau composite flexible avec une feuille plastique perforée collée à une couche fibreuse et procédé pour la fabrication de celui-ci

(30) Priority: 12.06.1989 DE 3919166
(43) Date of publication of application: 19.12.1990
(73) Proprietor: BP Chemicals PlasTec GmbH, 89165 Dietenheim (DE)
(72) Inventor: Utz, Kastulus, D-8201 Neubeuern (DE)
(74) Representative: Dost, Wolfgang, Dr.rer.nat., Dipl.-Chem.

(56) References cited:
- EP-A- 0 209 713
- DE-A- 1 504 799
- FR-A- 2 227 126
- GB-A- 2 014 508
- US-A- 3 459 618
- US-A- 3 523 149
- US-A- 3 881 489
- US-A- 3 989 867
- US-A- 4 364 723
- US-A- 4 550 546

## Description

The firm bonding of a perforated film to a fibre layer produces a surface material that has good properties as regards thermal insulation and the exclusion of liquid and dust. It may be used for clothing, especially protective clothing, or dressings and such like. The perforations should permit the interchange of air and moisture without allowing liquid to enter. Producing perforations that, on the one hand, are sufficiently narrow and, on the other, are sufficiently numerous to permit a satisfactory interchange of gas, is difficult. Such tiny perforations cannot be produced by mechanical means. Intentional tear formation in the film, produced by embossing pressure (DE-A 37 24 510) does not give a reproducible, constant perforation size. A proposal was therefore made (DE-A 15 04 709) to subsequently reduce the perforations produced in a film, to the desired extent, by closing them up. This is expensive and difficult, and results in an undesirably excessive consumption of film material. The effect is also very limited, a maximum of about 10% change in the permeability being achieved, which is not sufficient for reducing mechanically produced perforations to the required extent.

GB-A-2 014 508 discloses a composite structure, wherein an embossed film having perforations with openings in the embossments is bonded to a fibrous layer, wherein said composite structure allows a rapid transport of liquid through the embossed film but prevents a backflow of liquid from the absorbent layer through a kind of lip-valve action.

It is an object of the present invention to provide a flexible composite and a method for producing it by firmly bonding a perforated plastics film to a fibrous layer, by exerting pressure and heat, which reproducibly reduces the size of the perforations.

The solution according to the invention is defined in claims 1 and 5.

In pressing the embossments together, the edges of the perforations are also greatly deformed so that the size of the perforations is reduced. By irregular crushing of the embossments, it is possible to obtain a minimum reduction in size for each perforation within statistically reproducible limits.

The compression process is preferably carried out at right angles to the plane of the film, and in the course of bonding the film to the fibrous layer. This is done simply by bonding the film to the fibrous layer under pressure and heat since, in such a method the embossments are anyway compressed at right angles to the plane of the film. Since the bonding takes place under the action of heat, preferably in a welding process, the embossments are simultaneously fixed in the compressed state due to thermoplastic deformation. Moreover, due to their arrangement on one side of the film facing the fibrous layer, the embossments are also held in place, in the compressed state, by the fibrous layer.

The type of the fibre bonding in the fibrous layer is not critical to the invention but a non-woven fibrous layer is preferably used because (a) it can be easily made, (b) it has a more uniform structure than woven or knitted materials, and (c) in view of its ability to prevent ingress of water and dust, may be beneficial as regards the sealing property.

Thus the non-woven fibrous layer preferably consists of a material that can be welded to the film. The non-woven fibrous layer may consist of staple fibres, fibrous mixtures or spun-bonded fibres. They are of such a composition that fixing by thermal means is possible. The fibrous material can also be treated with antistatic, hydrophilic or hydrophobic, or flame retardant additives. Both film and fibrous layers may contain starch which enables biological degradation of the composite.

To produce a satisfactory bonding of the film and fibrous layers, their melting ranges should be very close. It has been shown that a co-extruded fibre consisting of a polypropylene core and an outer layer of polyethylene, in combination with a polyethylene film can be used very successfully. To enable purely non-woven fibrous layers of pure polypropylene to be used it is important that the melting point of the film is close to that of the polypropylene. It was found that mixtures of polypropylene and polyethylene - particularly low-density polyethylene - or mixtures of polypropylene and ethylene/vinyl acetate copolymer are most suitable.

With regard to the heat-bonding under pressure, a spot-weld type of bonding is preferred since this can be done at the same time that the internal reinforcement of the fibrous layer is carried out so that the fibres in the vicinity of these spot welding points are welded not only to the film but to each other. In so doing, the pattern of weld points in relation to the pattern of the perforations in the film should be such that these perforations are not closed to any great extent by the weld points. The pattern and dimensions of the weld points should not be allowed to coincide with those of the film perforations and should be mutually positioned so that the perforations in the film are not affected by the weld points. This can be achieved in practice, if the distance between the centres of the weld points, at least in one direction, differs from the distance between centres of the embossments and a simple multiple and a simple fraction of it, such that the weld points and film perforations overlap only by chance and in very limited areas, this overlapping being statistically uniform. Preferably, the distance between the bonding points in at least one direction is not more that twice the centre-to-centre distance of the embossment. It is also advisable for the bonding area of the weld points to be between 5 and 30% of the total area of the composite. The superficial extent of the weld points should be less than the largest dimension of the perforations so that complete closure of the perforations is improbable even if a weld point coincides with a perforation.

To produce a weld joint between the film and the fibrous layer, the film and fibre material(s) of the fibrous layer should have substantially the same or at least similar melting ranges.

According to the invention, the narrowing of the size of the perforations not only occurs due to irregular crushing of the embossments, as is likely to occur with compression in a direction at right angles to the film surface, but the perforations which previously have approximately the same cross-sectional dimensions (for example are bounded by a circle or a polygon) are stretched in one direction to form a slit. According to the invention, this is achieved by stretching the film in a longitudinal direction, whereby the perforations tend to narrow in the form of a slit, i.e. more so than that due to stretching alone, and additionally, if the compression takes place subsequently or sumultaneously at right angles to the film surface. The perforation boundaries that are opposite each other at right angles to the stretching direction fold together, as it were, over one another so that the perforation itself is then deformed into a narrow slit if, say, it was previously circular in shape. The extent of the stretching required can be easily determined by testing. In general, slight stretching which occurs anyway due to the longitudinal tension is sufficient if the film and the fibre layer are passed through a calender for bonding purposes.

To prevent perforations from being closed due to their edges over-lapping one another, provision is made to ensure that the wall length of the embossments, measured between the base of the embossments in the compressed film surface and the edge of the perforation, is not significantly greater than half of the base diameter of the embossments. The wall lengths must usually be slightly larger because the compression often results in a shortening of the wall length due to irregular bending or crushing.

The invention is further illustrated below by reference to the drawings accompanying the specification.
- Fig. 1: - the cross section of a suitable plastics film
- Fig. 2: - the cross section of another version of a suitable plastics film
- Fig. 3: - the cross section of the finished surface material
- Fig. 4: - an enlarged view from above of part of the film side of such a composite.

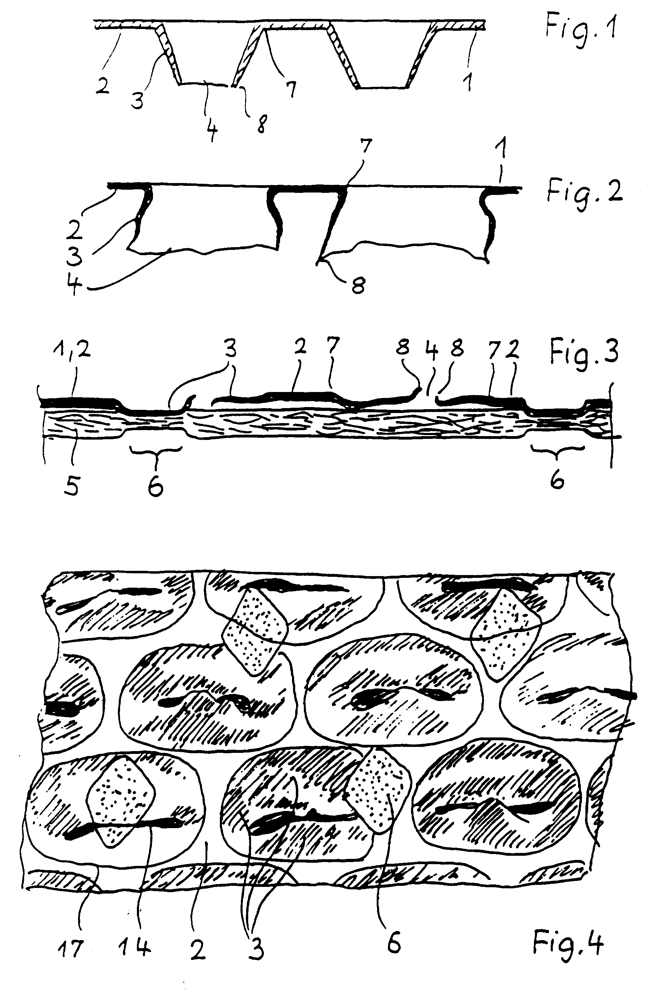

Figs. 1 and 2 show films that can be produced, for example, according to DE-A 29 21 078, DE-A 27 46 440, DE-C 25 56 501, EP-A 0 138 601, GB-A 2 014 508, EP-A 0 203 823, EP-A 0 141 654 or DE-A 37 23 404. Film 1 shows, projecting from the film surface 2, embossments whose walls 3, at least partly tapered towards the corresponding perforation, are specifically reduced by deformation and at their uppermost point contain an opening 4 which preferably is of uniform cross-section. The embossments and the perforations may however be stretched longitudinally or be oval shaped whereby, specifically, their longitudinal direction coincides with the elongation direction in the subsequent compression of the embossments. The dimensions of the perforations (the transverse dimensions in the case of the longitudinally stretched perforations) in the initial state are specifically of the order of one to a few tenths of a millimetre. The heights of the embossments above the uncompressed film surface 2 is between 0.2 and 3 times the perforation diameter, ideally 0.5 to 2 times. The wall length of the embossments, between the base 7 and the edge 8 of the perforation, is preferably not significantly more than half of the base diameter. The mid-point distance between the embossments is preferably one to three times the diameter of the base.

The film may be of uniform thermoplastic materials, mainly polyolefins, or blends of plastics, depending on the proposed application. The properties required, such as antistatic, feel, surface tension (water repellant), antibacterial or flame resistant properties can be produced by means of additives. Delustering material such as chalk, microtalcum, aluminium hydroxide or silicic acid, for example, may be added. The open area of the perforated film in its initial state constitutes suitably between 4 and 40%, preferably between 10 and 20% of the total area.

To reinforce the non-woven fibrous layer and to bond the film and fibrous layers, they are passed through a heated embossing calender as illustrated and explained, for example, in DE-A 37 24 510. The heated rolls of the embossing calender are so designed that spot welding occurs in the area of the points 6 (Fig. 4) which are uniformly distributed and whose individual area is in proportion to the size of the perforation walls 3 and the openings 4. The perforation walls 3 are compressed between the calender rolls at right angles to the film surface, whereby the opposite parts of their walls and edges come close together as shown in Fig. 3. This proximity of the opposite edges of the holes is required so that tension is exerted on the film at right angles to the film surface. By this means, the slit shape of the perforation 14 shown in Fig. 4 is obtained. This Figure 4 shows a 40 X electron micrograph of a sample in which opening slits 14 are shown black, and the shadows produced in the area of the irregularly compressed embossments are shown shaded. The base lines of the embossments are illustrated by lines 17. The undeformed area of the film between the embossments appears white. Weld points are shown as dots. It is seen that a relatively uniform, reproducible reduction in the size of the perforation to an average width can be achieved which is of the order of one tenth of the initial diameter, deviations at the top being comparatively rare. Depending on the size of the initial openings 4, the width of the opening slit 14 is a few hundredths to about one tenth of a millimetre. This is sufficient, for example in combination with conventional water repelling agents, to ensure prevention of water and dust passing therethrough, therefore rendering it suitable for clothing purposes.

During the processing of the film, involving stretching, no specific tension forces, or only slight tension forces, are exerted at right angles to the stretching direction, so that its dimension is retained in this transverse direction. Even under the effect of stretching, the size of the perforation can contract in the other direction. In some cases, it may even be of benefit to allow a slight formation of small corrugations in the stretching direction.

As can be seen from Fig. 4, the pattern of weld points 6 differs from that of the perforation walls 3 such that as a rule no adjacent perforation in the film is completely closed. The composite produced according to the invention may consist entirely of film and fibrous layers. Firm or loose bonding with other layers by this means however is not excluded.

### Example 1

A perforated film of approx. 32 g/m², consisting of linear low density polyethylene, with a density of 0.912 and a melt flow index of 3.3 was applied to a staple non-woven fibrous layer, having fibre fineness of 1.7 dtex (40 g/m²) and consisting of a two-component fibres, low density polyethylene/polypropylene, (LDPE/PP) before feeding into the thermal bonding calender. The perforated film had about 120 perforations/cm². The diameter of the perforations was approx. 0.32 mm. This corresponds to a free area of about 10% of the total area of the composite. The perforations were venturi-shaped embossments, whereby their free ends could face either towards the non-woven fibrous layer or away from it. The proportion of the bonding area produced by compressing in a steel roll was about 20% of the total area of the composite. The perforations were compacted in the weld area so that a total open area of about 6% was produced. The non-woven fibrous layer and the film were antistatically treated; the non-woven fibrous layer was also treated with a water repellant. Complete watertightness was achieved, particularly with respect to prevention of splashing. Due to the thorough bonding of the fibrous layer to the film, and the fineness of the perforations, the composite was virtually free from loose fibres, and was impermeable to dust; the composite could be used for protective coating in clean areas.

### Example 2

As in Example 1, a perforated film (32 g/m²) was applied (before bonding the non-woven fibrous layer) to a spun-bonded non-woven fibrous layer (40 g/m²) with a fibre fineness of 1.7 dtex, and consisting of pure polypropylene (PP). The film, consisted of 40 parts of a PP, density 0.898, melt flow index 8, and 60 parts of a linear low density polyethylene (LLDPE) of density 0.912, melt flow index 3.3. The non-woven fibrous layer and film had received prior antistatic and water repellant treatment. The film material had a melting point of about 150°C. This melting point was sufficiently close to that of the PP to be within a common melting range on sealing the film with the non-woven fibrous layer to avoid burning through the film. Due to the high proportion of LLDPE of low density, the required softness and elasticity of the whole composite was achieved. In order to obtain a dry feel and a matt surface, about 10% of chalk (particle size from about 1 to 3 micron) in concentrate form was added to the film material.

### Example 3

A perforated film, which was also treated with a flame retardant, was welded to a non-woven fibrous layer (corresponding to Example 1) as described in Examples 1 and 2, during the bonding of the non-woven fibrous layer. The flame retardant property of the film was achieved by adding commercial concentrates, ie 15% of the concentrate B 10318 (made by Constab) was added to the LLDPE type (density: 0.912, MFI: 3.3). The non-woven fibrous layer consisted of staple fibres (40 g/m²). The film had a weight of approx. 32 g/m². The bond complied with the flame retardant standard according to DIN 53438 both as regards edge flaming (K1) and surface flaming (F1) of the front and back.

## Claims

1. A method of preparing a flexible composite, wherein an embossed plastics film (1) having perforations with openings (4) in the embossments is bonded to a fibrous layer (5), said perforations with openings (4) facing said fibrous layer (5)
**characterized** in that said bonding is carried out by heat-bonding under pressure, permanently compressing the composite to a structure wherein the opposite edges (8) of the perforation walls (3) come into proximity resulting in narrowed openings (4), wherein the film (1) is stretched in longitudinal direction and the compression takes place subsequently or simultaneously at right angles to the film surface and wherein the length of said perforation walls (3), measured between the base (7) of the embossments and the edge (8) of the perforations, is not significantly greater than half the base diameter of the embossments.

2. The method of claim 1, wherein the perforation walls (3), which are at least partially conical in shape, narrow towards the corresponding openings (4).

3. The method of claim 1 or 2, wherein the film (1) and the fibrous layer (5) are bonded by spot welding (6).

4. The method of claim 3, wherein said spot welding is carried out with simultaneous bonding of the fibrous layer (5) to reinforce said layer.

5. A flexible composite comprising an embossed plastics film (1) having perforations with openings (4), and a fibrous layer (5) firmly bonded to each other, said perforations with openings (4) facing said fibrous layer (5), **characterized** in that said composite is in permanently compressed state having the opposite edges (8) of the perforation walls (3) in proximity with narrowed openings (4) compared to the uncompressed state, wherein said openings (4) are narrowed to longitudinal slits (14) along the length of the film (1) and the film (1) and the fibrous layer (5) are spot welded (6).

6. The composite of claim 5, wherein the fibrous layer (5) is a non-woven layer bonded in itself by weld points (6).

7. The composite of claim 5 6, wherein the melting points of the materials forming the fibrous layer (5) and the film (1) are close together.

8. The composite of any of claims 5 - 7, wherein the bonding area of the weld points (6) occupies between 5 to 30 % of the total surface area of the composite.

9. The composite of any of claims 5 - 8, wherein the distance between the weld points (6) in at least one direction is not greater than double the mid-point distance between the embossments.

## Patentansprüche

1. Verfahren zur Herstellung eines flexiblen Verbundmaterials, bei dem eine geprägte Kunststoffolie (1), die Perforationen mit Öffnungen (4) in den Prägungen aufweist, mit einer Faserschicht (5) verklebt ist, wobei die Perforationen mit den Öffnungen (4) der Faserschicht (5) zugewandt sind, dadurch gekennzeichnet, daß die Verklebung durch eine Wärmeverklebung unter Druck durchgeführt wird, wobei das Verbundmaterial permanent zu einer Struktur zusammengedrückt wird, bei der die gegenüberliegenden Kanten (8) der Perforationswände (3) einander nahekommen, so daß sich verengte Öffnungen (4) ergeben, wobei die Folie (1) in Längsrichtung gestreckt wird und das Zusammendrücken nachher oder gleichzeitig rechtwinkelig zur Folienoberfläche stattfindet, und wobei die Länge der Perforationswände (3), gemessen zwischen der Basis (7) der Prägungen und der Kante (8) der Perforationen, nicht signifikant größer als die Hälfte des Basisdurchmessers der Prägungen ist.

2. Verfahren nach Anspruch 1, wobei die Perforationswände (3), die zumindest teilweise konisch geformt sind, sich in Richtung zu den entsprechenden Öffnungen (4) verengen.

3. Verfahren nach Anspruch 1 oder 2, wobei die Folie (1) und die Faserschicht (5) durch Punktschweißen (6) verklebt sind.

4. Verfahren nach Anspruch 3, wobei das Punktschweißen unter gleichzeitigem Verkleben der Faserschicht (5) zur Verstärkung dieser Schicht durchgeführt wird.

5. Flexibles Verbundmaterial, umfassend eine geprägte Kunststoffolie (1) mit Perforationen mit Öffnungen (4) und eine Faserschicht (5), die fest miteinander verklebt sind, wobei die Perforationen mit Öffnungen (4) der Faserschicht (5) zugewandt sind, dadurch gekennzeichnet, daß sich das Verbundmaterial in einem permanent zusammengedruckten Zustand befindet, wobei die gegenüberliegenden Kanten (8) der Perforationswände (3) sich im Vergleich zum nicht zusammengedrückten Zustand nahe bei den verengten Öffnungen (4) befinden, wobei die Öffnungen (4) entlang der Länge der Folie (1) zu länglichen Schlitzen (14) verengt sind und die Folie (1) und die Faserschicht (5) durch Punktschweißen (6) verbunden sind.

6. Verbundmaterial nach Anspruch 5, wobei es sich bei der Faserschicht (5) um eine Vliesschicht handelt, die in sich selbst durch Schweißpunkte (6) verklebt ist.

7. Verbundmaterial nach Anspruch 5 oder 6, wobei die Schmelzpunkte der Materialien zur Bildung der Faserschicht (5) und der Folie (1) nahe beeinander liegen.

8. Verbundmaterial nach einem der Ansprüche 5 bis 7, wobei die Verklebungsfläche der Schweißpunkte (6) zwischen 5 bis 30% der Gesamtoberfläche des Verbundmaterials ausmacht.

9. Verbundmaterial nach einem der Ansprüche 5 bis 8, wobei der Abstand zwischen den Schweißpunkten (6) in mindestens einer Richtung nicht mehr als das Doppelte des Mittelpunkt-Abstands zwischen den Prägungen beträgt.

## Revendications

1. Un procédé de préparation d'un composite souple, où l'on colle à une couche fibreuse (5) un film en plastique gaufré (1) comportant des perforations avec des ouvertures (4) dans les gaufrages, les perforations avec les ouvertures (4) étant tournées vers la couche fibreuse (5),
caractérisé en ce que le collage est effectué par thermocollage sous pression, comprimant de façon permanente le composite en une structure dans laquelle les bords opposés (8) des parois (3) des perforations sont amenés à proximité, donnant des ouvertures rétrécies (4),
dans lequel le film (1) est étiré dans le sens longitudinal et la compression a lieu subséquemment ou simultanément à angle droit de la surface du film,
et dans lequel la longueur des parois (3) des perforations, mesurée entre la base (7) des gaufrages et le bord (8) des perforations, n'est pas significativement supérieure à la moitié du diamètre de base des gaufrages.

2. Le procédé de la revendication 1, dans lequel les parois (3) des perforations, qui ont une forme au moins partiellement conique, se rétrécissent en direction des ouvertures correspondantes (4).

3. Le procédé de la revendication 1 ou 2, dans lequel le film (1) et la couche fibreuse (5) sont collés par soudage par points (6).

4. Le procédé de la revendication 3, dans lequel le soudage par points est effectué avec collage simultané de la couche fibreuse (5) pour renforcer cette couche.

5. Un composite souple comprenant un film gaufré en plastique (1) pourvu de perforations avec des ouvertures (4), et une couche fibreuse (5) collés de façon résistante entre eux, les perforations avec les ouvertures (4) étant tournées vers la couche fibreuse (5), caractérisé en ce que ce composite est à l'état comprimé en permanence avec les bords opposés (8) des parois (3) des perforations à proximité avec des ouvertures rétrécies (4) par rapport à l'état non comprimé, ces ouvertures (4) étant rétrécies en fentes longitudinales (14) suivant la longueur du film (1) et le film (1) et la couche fibreuse (7) sont soudés par points (6).

6. Le composite de la revendication 5, dans lequel la couche fibreuse (5) est une couche non tissée collée en elle-même par des points de soudage (6).

7. Le composite de la revendication 5 ou 6, dans lequel les points de fusion des matériaux formant la couche fibreuse (5) et le film (1) sont proches entre eux.

8. Le composite de l'une des revendications 5 à 7, dans lequel la surface de collage des points de soudage (6) occupe entre 5 et 30 % de l'étendue de surface totale du composite.

9. Le composite de l'une des revendications 5 à 8, dans lequel la distance entre les points de soudage (6) dans au moins une direction n'est pas supérieure à deux fois la distance des points milieux entre les gaufrages.
